(19) Europäisches Patentamt
European Patent Office
Office européen des brevets

(11) Publication number: **0 003 016 B1**

# (12) EUROPEAN PATENT SPECIFICATION

(45) Date of publication of patent specification: 30.12.81

(21) Application number: 78101172.1

(22) Date of filing: 18.10.78

(51) Int. Cl.³: C 07 D 498/04, C 07 D 513/04, A 61 K 31/55 //(C07D498/04, 241/00, 267/00), (C07D513/04, 241/00, 281/00)

## (54) Pyrazino-benzoxazepine and -benzthiazepine derivatives, processes for their production and pharmaceutical compositions containing them.

(30) Priority: 31.10.77 CH 13219/77
08.06.78 CH 6281/78

(43) Date of publication of application:
25.07.79 Bulletin 79/15

(45) Publication of the grant of the European patent:
30.12.81 Bulletin 81/52

(84) Designated Contracting States:
BE CH DE FR GB LU NL SE

(56) References cited:
DE - A - 1 595 897
DE - A - 2 123 784
US - A - 3 459 737

(73) Proprietor: SANDOZ AG
Lichtstrasse 35
CH-4002 Basel (CH)

(72) Inventor: Leutwiler, Albert, Dr.
Kappelenring 40d
CH-3032 Hinterkappelen (CH)
Inventor: Sorg, Dieter, Dr.
Wylerstrasse 11
CH-3014 Berne (CH)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 003 016 B1

Pyrazino-benzoxazepine and -benzothiazepine derivatives, processes for their production and pharmaceutical compositions containing them.

The present invention relates to pyrazino-benzoxazepines, and -benzothiazepines, processes for their production and pharmaceutical compositions containing them.

The present invention provides a compound of formula I,

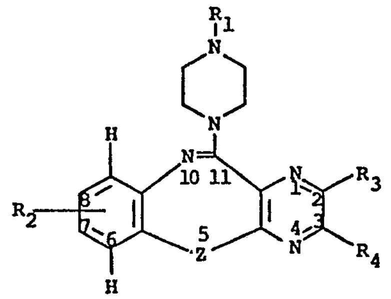

I

wherein $R_1$ is hydrogen, alkyl of 1 to 4 carbon atoms, hydroxyalkyl with a maximum of 4 carbon atoms, which may be acylated by an alkanoyl group of 2 to 18 carbon atoms, alkoxyalkyl with a maximum of 6 carbon atoms, cycloalkyl of 3 to 6 carbon atoms, cycloalkylalkyl of 4 to 7 carbon atoms or a group of formula II,

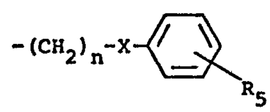

II

wherein $R_5$ is hydrogen, halogen, alkyl or alkoxy of 1 to 4 carbon atoms, and
either i) X is $-CH_2-$ and
n is 0, 1, 2 or 3 or
ii) X is —CO— and
n is 1, 2 or 3 or
iii) X is —O— and
n is 2 or 3

and $R_2$ and $R_3$ are independently hydrogen, halogen, trifluoromethyl or alkyl, alkoxy, alkylthio, alkylsulfinyl or alkylsulfonyl, each of 1 to 4 carbon atoms and
$R_4$ is hydrogen, alkyl or alkoxy of 1 to 4 carbon atoms and
Z is —O— or —S—,
with the proviso that, when $R_3$ is trifluoromethyl, alkoxy, alkylthio, alkylsulfinyl or alkylsulfonyl, $R_4$ is other than alkoxy, or an acid addition salt thereof.

Any alkyl, alkoxy or alkylthio radical of 1 to 4 carbon atoms is preferably of 1 to 3 carbon atoms, especially 1 or 2 carbon atoms. Hydroxyalkyl has preferably 2 or 3 carbon atoms. Preferably the hydroxy group is attached to a carbon atom other than the carbon atom adjacent to the nitrogen atom. The alkoxy moiety in alkoxyalkyl is preferably located in the terminal position of the alkylene chain which preferably has 2 or 3, especially 2 carbon atoms. The alkoxy radical in alkoxyalkyl is preferably methoxy. Cycloalkyl or the cycloalkyl moiety of cycloalkylalkyl is conveniently cyclopropyl or cyclopentyl. The alkyl moiety of cycloalkylalkyl is conveniently methyl. Halogen means fluorine, chlorine, bromine or iodine, preferably fluorine or chlorine, especially chlorine.

$R_1$ is preferably hydrogen, alkyl or a group of formula II. $R_5$ is preferably hydrogen or fluorine. X is preferably $-CH_2-$ or —CO—. When X is —O—, n is preferably 3 or when X is $-CH_2-$ or —CO— n is preferably 1 or 3. $R_2$ is preferably hydrogen, halogen or alkoxy. $R_3$ is preferably hydrogen, halogen or alkyl. $R_4$ is preferably hydrogen. When $R_2$ and $R_3$ are other than hydrogen, $R_2$ is preferably in position 8. Z is preferably —O—.

The present invention also provides a process for the production of a compound of formula I as defined above, which comprises reacting a compound of formula III,

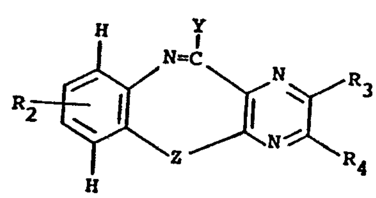

III

wherein $R_2$, $R_3$, $R_4$ and Z are as defined above and
Y is a leaving group,
with a compound of formula IV,

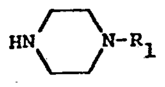 IV

wherein R1 is as defined above.

The reaction may be effected in conventional manner for the production of similar compounds. Y is preferably chlorine.

The process may be conveniently effected at a temperature of from 20 to 170°C in an inert organic solvent such as toluene, methylene, chloride or dioxane. When $R_1$ is hydrogen conveniently a compound of formula III is added to a solution of a compound of formula IV.

The starting material of formula III may be prepared in known manner, e.g. as described herein, for example via the corresponding lactam, e.g. by reaction with phosphoroxychloride.

Insofar as the production of starting materials is not particularly described these compounds are known or may be produced in analogous manner to known compounds.

Free base forms of the compounds of formula I may be converted into acid addition salt forms in conventional manner and vice versa. Suitable acids are e.g. maleic acid, oxalic acid, methanesulphonic acid, hydrochloric acid and hydrobromic acid.

In the following Examples the temperatures given are in degrees Centigrade and are uncorrected. In the table the following abbreviations are used:

*) monomaleate
**) monooxalate

Example 1

11-(4-Methyl-1-piperazinyl)-pyrazino[2,3-b][1,5]benzoxazepine

3.6 g 1-methylpiperazine are added to a stirred suspension of 4.2 g 11-chloro-pyrazino[2,3-b][1,5]benzoxazepine in 50 ml abs. toluene. Stirring is maintained for four hours at room temperature. 100 ml water and 100 ml ethyl acetate are added and the mixture well shaken. The organic phase is filtered, dried over anhydrous magnesium sulphate and evaporated. The residue is dissolved in ethanol and 1 equivalent of maleic acid in ethanol is added, to yield the heading compound in monomaleate salt form, m.p. 190—191°.

The starting material 11-chloro-pyrazino[2,3-b] [1,5]benoxazepine may be obtained as follows:

a) *pyrazino[2,3-b][1,5]benzoxazepin-11(10H)-one*

A solution of 51 g of 3-bromo-pyrazine-2-carboxylic acid in 130 ml hexamethylphosphorotriamide is cooled to —10° and treated under stirring dropwise with 18 ml of thionyl chloride. After 5 minutes there are added in one portion 27.3 g of o-aminophenol and the reaction mixture is stirred for 2 hours at room temperature. The mixture is poured on ice and extracted with ethyl acetate. The organic layer is washed with 2N hydrochloric acid, 2N sodium carbonate and finally with water, dried over anhydrous magnesium sulphate, filtered and evaporated. The residue is treated with 2.2 l of 0.1 N sodium hydroxide and stirred at 70° for 15 hours. The resulting solid is filtered, washed with water and dried in vacuo at 80° to yield the heading compound, m.p. 269—272°.

b) *11-chloro-pyrazino[2,3-b][1,5]benozoxazepine*

16 g of pyrazino[2,3-b][1,5]benzoxazepine-11-(10H)-one, 140 ml of phosphoroxychloride and 5.6 ml of N,N-dimethylaniline are refluxed for 15 hours. Residual phosphoroxychloride is removed by distillation. The residue is dissolved in methylene chloride and poured on ice. The methylene chloride layer is washed with 4N hydrochloric acid and water, dried over anhydrous magnesium sulphate and evaporated to give the heading compound, m.p. 123—126°.

In analogous manner to that described in Example 1, the following compounds of formula I are obtained, wherein Z is —O—:

| Example | $R_1$ | $R_2$ | $R_3$ | $R_4$ | m.p. |
|---|---|---|---|---|---|
| 2 | H | H | H | H | 223–224** |
| 3 | $CH_2CH_2OH$ | H | H | H | 218–220** (dec.) |
| 4 | H | 8–Cl | H | H | 197–199* (dec.) |
| 5 | $CH_3$ | 8–Cl | H | H | 118–119 |
| 6 | $CH_2CH_2OH$ | 8–Cl | H | H | 230–232** (dec.) |
| 7 | $CH_3$ | H | $CH_3$ | H | 171–173* |
| 8 | $CH_3$ | H | $CH_3$ | $CH_3$ | 175–177* |
| 9 | $CH_3$ | H | Cl | H | 134–135 |
| 10 | H | H | H | $CH_3$ | 197–198* |
| 11 | H | H | $CH_3$ | H | 178–180* |
| 12 | H | H | Cl | H | 184–186* |
| 13 | $C_2H_5$ | H | H | H | 170–171* |
| 14 | $CH(CH_3)_2$ | H | H | H | 145–148* |
| 15 | $CH_2CH_2OCH_3$ | H | H | H | 157–158* |
| 16 | H | 8–$CH_3$ | H | H | 196–197* |
| 17 | $CH_3$ | 8–$CH_3$ | H | H | 171–172* |
| 18 | H | 7–$CH_3$ | H | H | 215–216* |
| 19 | $CH_3$ | 7–$CH_3$ | H | H | 160–162* |
| 20 | H | 8–$OCH_3$ | H | H | 178–180* |
| 21 | $CH_3$ | 8–$OCH_3$ | H | H | 159–161* |
| 22 | H | 8–F | H | H | 181–182* |
| 23 | $CH_3$ | 8–F | H | H | 170* |
| 24 | $CH_3$ | H | H | $CH_3$ | 178–179 |
| 25 | $CH_2CH_2$ 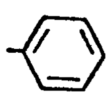 | H | H | H | 140–141 |
| 26 | $CH_2CH_2$ 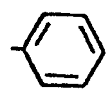 | 8–$OCH_3$ | H | H | 215–216** (dec.) |
| 27 | $CH_2CH_2$ 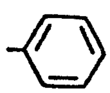 | H | H | $CH_3$ | 201–203 |
| 27a | $CH_2CH_2$ 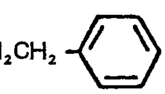 | H | Cl | $CH_3$ | 204–208* |

| Example | $R_1$ | $R_2$ | $R_3$ | $R_4$ | m.p. |
|---|---|---|---|---|---|
| 28 | $CH_2CH_2$ 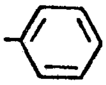 | H | $CH_3$ | H | 136–138 |
| 29 | $(CH_2)_2O$ 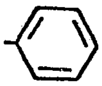 | H | H | H | 160–162** (dec.) |
| 30 | $(CH_2)_3CO$ 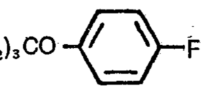 | H | H | H | 130–131 |
| 31 | $CH_2CH_2$ 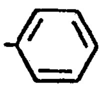 | H | Cl | H | 135–137 |
| 32 | $CH_2$ 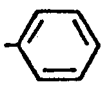 | H | H | H | 137–138* |
| 33 | $CH_2CH_2$ 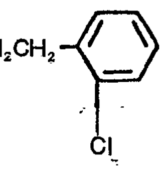 | H | H | H | 119–121 |
| 34 | $(CH_2)_2CH_2$ 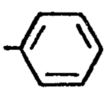 | H | H | H | 133–135** (dec.) |
| 35 | H | H | $CH_3$ | $CH_3$ | 200–202* |
| 36 | $CH_2CH_2$ 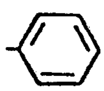 | H | $CH_3$ | $CH_3$ | 182–183 |
| 37 | H | 8–Cl | $CH_3$ | H | 192–193* |
| 38 | $CH_3$ | 8–Cl | $CH_3$ | H | 189–192* |
| 39 | $CH_2CH_2$ 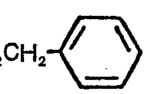 | 8–Cl | $CH_3$ | H | 212–213** (dec.) |
| 40 | $CH_3$ | 8–Cl | Cl | H | 207–209 |
| 41 | H | H | $OCH_3$ | H | |
| 42 | $CH_2CH_2$ 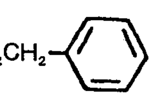 | 8–Cl | H | H | |

| Example | $R_1$ | $R_2$ | $R_3$ | $R_4$ | m.p. |
|---|---|---|---|---|---|
| 43 | $CH_2$- 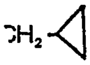 | H | H | H | |
| 44 | $CH_2$- 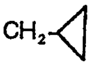 | H | Cl | H | |
| 45 | $CH_2$- 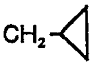 | 8–Cl | H | H | |
| 46 | 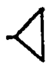 | H | H | H | |
| 47 | 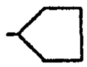 | H | H | H | |
| 48 | $CH_3$ | H | $OCH_3$ | H | |
| 49 | $CH_2CH_2$- 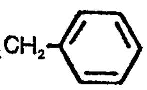 | H | $OCH_3$ | H | |
| 50 | H | H | Cl | $CH_3$ | |
| 51 | $CH_3$ | H | Cl | $CH_3$ | 121–123* |
| 52 | $CH_3$ | H | $SCH_3$ | H | |
| 53 | $CH_3$ | H | $SOCH_3$ | H | |
| 54 | $CH_3$ | H | $SO_2CH_3$ | H | |

In analogous manner to that described in Example 1, the following compounds of formula I are obtained, wherein Z is —S—:

| Example | $R_1$ | $R_2$ | $R_3$ | $R_4$ | m.p. |
|---|---|---|---|---|---|
| 55 | H | H | H | H | 158–161 |
| 56 | $CH_3$ | H | H | H | 171–173* |
| 57 | H | 8–Cl | H | H | 191–192* (dec.) |
| 58 | $CH_3$ | 8–Cl | H | H | 148–149 |
| 59 | $CH_2CH_2OH$ | 8–Cl | H | H | 235–237** (dec.) |
| 60 | $CH_2CH_2OH$ | H | H | H | 253–256 naphthalene-1,5-disulphonate (dec.) |
| 61 | $CH_3$ | 7–Cl | H | H | 169–170 |
| 62 | H | 7–Cl | H | H | 209–211* |
| 63 | $CH_3$ | 8–F | H | H | |
| 64 | H | 8–F | H | H | |
| 65 | $CH_3$ | 8–F | $CH_3$ | H | |
| 66 | $CH_3$ | 8–F | Cl | H | |
| 67 | $CH_3$ | H | $CH_3$ | H | |
| 68 | $CH_3$ | H | Cl | H | |
| 69 | $CH_3$ | H | $SCH_3$ | H | |
| 70 | $CH_3$ | H | $SOCH_3$ | H | |
| 71 | $CH_3$ | H | $SO_2CH_3$ | H | |
| 72 | $CH_3$ | 8–Cl | $CH_3$ | H | |
| 73 | H | 8–Cl | $CH_3$ | H | |
| 74 | $CH_3$ | 8–Cl | Cl | H | |

The starting material 11-chloro-pyrazino[2,3-b][1,5]benzothiazepine for Example 55 may be prepared as follows:

a) *3-(2-amino-phenylthio)-pyrazine-2-carboxylic acid methyl ester*

A mixture of 18 g 3-bromopyrazine-2-carboxylic acid methyl ester, 13.4 g 2-amino-thiophenolhydrochloride and 250 ml triethylamine is refluxed for 3 hours. Thereafter the solvent is evaporated and the residue treated with methylene chloride and water. The organic phase is dried over sodium sulphate and evaporated, whereby there is obtained the heading compound, which after recrystallisation from ethyl acetate has a m.p. of 155—156°.

b) *pyrazino[2,3-b][1,5]benzothiazepin-11(10H)-one*

Freshly prepared sodium methylate (from 1.8 g of sodium) in 200 ml abs. toluene and 16.8 g 3-(2-amino-phenylthio)-pyrazine-2-carboxylic acid methyl ester are stirred together at room temperature for 15 hours. The mixture is poured on ice and the resulting precipitate filtered, whereby there is obtained the heading compound, which after recrystallisation from ethyl acetate has a m.p. of 280—282°.

c) *11-chloro-pyrazino[2,3-b][1,5]benzothiazepine*

In analogous manner to that described in Example 1b) there is obtained the heading compound.

The compounds of formula I exhibit pharmacological activity. In particular, they exhibit sleep

inducing activity, as indicated in standard tests. For example in one test according to the principles of A. C. Sayers and G. Stille, Electroenceph. Clin. Neurophysiol. *27*, 87—89 (1969), the sleep inducing activity in rats is observed after a single peroral administration of from about 0,5 to about 80 mg/kg animal body weight.

The compounds are therefore indicated for use as sleep inducing agents. For this use, an indicated daily dose is from about 1 to about 100 mg conveniently given shortly before retiring to sleep.

Additionally, the compounds of formula I exhibit neuroleptic activity, as indicated in standard tests. For example, in one standard test an inhibition of spontaneous motor activity is observed in mice on p.o. administration of from 1 to 50 mg/kg animal body weight of the compounds in accordance with the principles of Caviezel and Baillod [Pharm. Acta Helv. (1958), *33*, 465—484).

The compounds are therefore indicated for use as neuroleptic agents. For this use an indicated daily dose is from about 50 to about 500 mg, conveniently given in divided doses 2 to 4 times a day in unit dosage form containing from about 12.5 to about 250 mg, or in sustained release form.

Additionally, the compounds of formula I exhibit antidepressant activity, as indicated in standard tests, for example, by an inhibition of tetrabenazine-induced catalepsy and ptosis in rats on intraperitoneal administration of from 5 to 15 mg/kg animal body weight in accordance with the method described by Stille (Arzneimittel-Forsch. 1964, *14*, 534).

The compounds are therefore indicated for use as antidepressant agents. For this use an indicated daily dose is from about 5 to about 150 mg, conveniently administered in divided doses 2 to 4 times a day in unit dosage form containing from about 1.25 to 75 mg, or in sustained release form.

The compounds of formula I may be administered in pharmaceutically acceptable acid addition salt form. Such acid addition salt forms exhibit the same order of activity as the free base forms. The present invention also provides a pharmaceutical composition comprising a compound of formula I, in free base form or in pharmaceutically acceptable acid addition salt form, in association with a pharmaceutical carrier or diluent. Such compositions may be in the form of, for example, a solution or a tablet.

The sleep inducing activity and the neuroleptic acitivity is the preferred utility for compounds of formula I. The compounds of formula I wherein Z is —O—, especially those of Examples 1 and 25 are especially indicated as sleep inducing agents. The compounds of Examples 5, 7, 9, 31, 38, 58 and 68 are especially indicated as neuroleptic agents.

In one group of compounds $R_1$ is hydrogen, alkyl of 1 to 4 carbon atoms, hydroxyalkyl with a maximum of 4 carbon atoms or alkoxyalkyl with a maximum of 6 carbon atoms, $R_2$ is hydrogen, halogen, alkyl or alkoxy of 1 to 4 carbon atoms, $R_3$ and $R_4$ are hydrogen and Z is —O—.

In another group of compounds $R_1$ is hydrogen, alkyl of 1 to 4 carbon atoms, hydroxyalkyl with a maximum of 4 carbon atoms, alkoxyalkyl with a maximum of 6 carbon atoms or a group of formula II, wherein $R_5$ is hydrogen, halogen, alkyl or alkoxy of 1 to 4 carbon atoms, and either i) X is —$CH_2$— and n is 0, 1, 2 or 3 or ii) X is —CO— and n is 1, 2 or 3 or iii) X is —O— and n is 2 or 3, $R_2$ is hydrogen, halogen, alkyl or alkoxy of 1 to 4 carbon atoms, $R_3$ is hydrogen, halogen, trifluoromethyl or alkyl, alkoxy or alkylthio, each of 1 to 4 carbon atoms, $R_4$ is hydrogen, alkyl or alkoxy of 1 to 4 carbon atoms and Z is —O—, with the proviso that when $R_3$ is trifluoromethyl, alkoxy or alkylthio, $R_4$ is other than alkoxy.

## Claims

1. A compound of formula I,

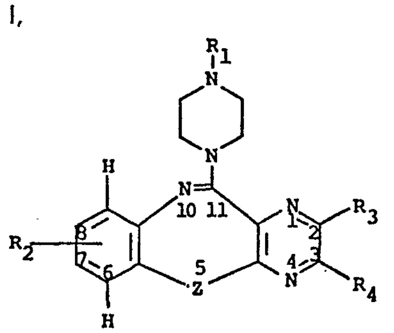

I

wherein $R_1$ is hydrogen, alkyl of 1 to 4 carbon atoms, hydroxyalkyl with a maximum of 4 carbon atoms, which may be acylated by an alkanoyl group of 2 to 18 carbon atoms, akoxyalkyl with a maximum of 6 carbon atoms, cycloalkyl of 3 to 6 carbon atoms, cycloalkylalkyl of 4 to 7 carbon atoms or a group of formula II,

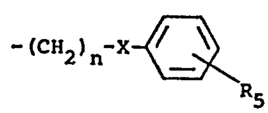

II

wherein $R_5$ is hydrogen, halogen, alkyl or alkoxy of 1 to 4 carbon atoms, and
either i) X is —$CH_2$— and
n is 0, 1, 2 or 3 or
ii) X is —CO— and
n is 1, 2 or 3 or
iii) X is —O— and
n is 2 or 3

and $R_2$ and $R_3$ are independently hydrogen, halogen, trifluoromethyl or alkyl, alkoxy, alkylthio, alkylsulfinyl or alkylsulfonyl, each of 1 to 4 carbon atoms and
$R_4$ is hydrogen, alkyl or alkoxy of 1 to 4 carbon atoms,
Z is —O— or —S—,
with the proviso that, when $R_3$ is trifluoromethyl, alkoxy, alkylthio, alkylsulfinyl or alkylsulfonyl, $R_4$ is other than alkoxy,

in the form of the free base or of an acid addition salt thereof.

2. A compound of claim 1, wherein Z is —O— in free base form or in acid addition salt form.

3. A compound of claim 1, wherein $R_1$ is hydrogen, alkyl of 1 to 4 carbon atoms, hydroxyalkyl with a maximum of 4 carbon atoms, alkoxyalkyl with a maximum of 6 carbon atoms or a group of formula II, wherein $R_5$ is hydrogen, halogen, alkyl or alkoxy of 1 to 4 carbon atoms, and either i) X is —$CH_2$— and n is 0, 1, 2 or 3 or ii) X is —CO— and n is 1, 2 or 3 or iii) X is —O— and n is 2 or 3, $R_2$ is hydrogen, halogen, alkyl or alkoxy of 1 to 4 carbon atoms, $R_3$ is hydrogen, halogen, trifluoromethyl or alkyl, alkoxy or alkylthio, each of 1 to 4 carbon atoms, $R_4$ is hydrogen, alkyl or alkoxy of 1 to 4 carbon atoms and Z is —O—, with the proviso that, when $R_3$ is trifluoromethyl, alkoxy or alkylthio, $R_4$ is other than alkoxy, in free base form or in acid addition salt form.

4. A compound of any one of claims 1, 2 and 3, wherein $R_1$ is hydrogen, alkyl or a group of formula II, in free base form or in acid addition salt form.

5. A compound of any one of claims 1 to 4, wherein $R_2$ is hydrogen, halogen or alkoxy, in free base form or in acid addition salt form.

6. A compound of any one of claims 1 to 5, wherein $R_3$ is hydrogen, halogen or alkyl, in free base form or in acid addition salt form.

7. A compound of any one of claims 1 to 6, wherein $R_4$ is hydrogen, in free base form or in acid addition salt form.

8. A compound of claim 1, wherein $R_1$ is hydrogen, alkyl of 1 to 4 carbon atoms, hydroxyalkyl with a maximum of 4 carbon atoms or alkoxyalkyl with a maximum of 6 carbon atoms, $R_2$ is hydrogen, halogen, alkyl or alkoxy of 1 to 4 carbon atoms, $R_3$ and $R_4$ are hydrogen and Z is —O—, in free base form or in acid addition salt form.

9. A compound of claim 1, which is 11-(4-methyl-1-piperazinyl)-pyrazino[2,3-b][1,5]benzoxazepine, in free base form or in acid addition salt form.

10. A compound of claim 1, which is 8-chloro-11-(4-methyl-1-piperazinyl)-pyrazino[2,3-b][1,5]benzoxazepine, 2-chloro-11-(4-methyl-1-piperazinyl)-pyrazino[2,3-b][1,5]benzoxazepine, 8-fluoro-11-(4-methyl-1-piperazinyl)-pyrazino[2,3-b][1,5]benzoxazepine, 3-methyl-11-(4-methyl-1-piperazinyl)-pyrazino[2,3-b][1,5]benzoxazepine, 8-chloro-2-methyl-11-(4-methyl-1-piperazinyl)-pyrazino[2,3-b][1,5]benzoxazepine or 2,8-dichloro-11-(4-methyl-1-piperazinyl)-pyrazino[2,3-b][1,5]-benzoxazepine, in free base form or in acid addition salt form.

11. A pharmaceutical composition comprising a compound of any one of claims 1 to 10 in free base form or in pharmaceutically acceptable acid addition salt form in association with a pharmaceutical carrier or diluent.

12. A compound as claimed in any one of claims 1 to 10, in free base form or in pharmaceutically acceptable acid addition salt form, for use in a method for the treatment of the human or animal body by therapy.

13. A compound as claimed in any one of claims 1 to 10, in free base form or in pharmaceutically acceptable acid addition salt form, for use in inducing sleep.

14. A process for the production of a compound of formula I, as defined in claim 1, which comprises reacting a compound of formula III,

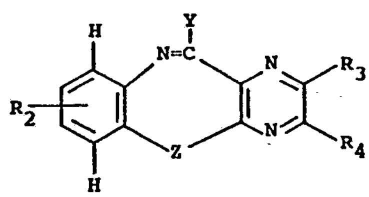

III

wherein $R_2$, $R_3$, $R_4$ and Z are as defined in claim 1 and
Y is a leaving group,

with a compound of formula IV,

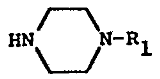

IV

wherein R1 is as defined in claim 1, and recovering the resultant compound in form of the free base or acid addition salt.

**Revendications**

1. Un composé de formule I

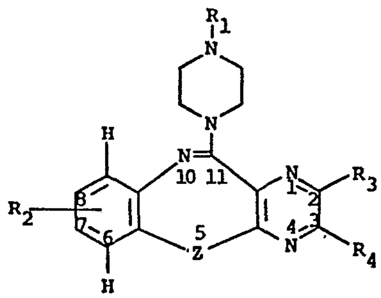

(I)

dans laquelle

$R_1$ signifie l'hydrogène, un groupe alkyle contenant de 1 à 4 atomes de carbone, hydroxyalkyle contenant au maximum 4 atomes de carbone, susceptible d'être acylé par un groupe alcanoyle contenant de 2 à 18 atomes de carbone, alcoxyalkyle contenant au maximum 6 atomes de carbone, cycloalkyle contenant de 3 à 6 atomes de carbone, cycloalkylalkyle contenant de 4 à 7 atomes de carbone ou un groupe de formule II

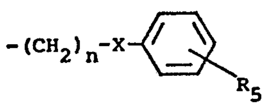

(II)

où $R_5$ signifie l'hydrogène, un halogène, un groupe alkyle ou alcoxy contenant de 1 à 4 atomes de carbone, et

soit i) X représente —$CH_2$— et n signifie 0, 1, 2 ou 3,

soit ii) X représente —CO— et n signifie 1, 2 ou 3,

soit iii) X représente —O— et n signifie 2 ou 3

et $R_2$ et $R_3$ représentent indépendamment l'hydrogène, un halogène, un groupe trifluorométhyle ou alkyle, alcoxy, alkylthio, alkylsulfinyle ou alkylsulfonyle, contenant chacun de 1 à 4 atomes de carbone, et $R_4$ signifie l'hydrogène, un groupe alkyle ou alcoxy contenant de 1 à 4 atomes de carbone, Z signifie —O— ou —S—, avec la condition que, lorsque $R_3$ signifie un groupe trifluorométhyle, alcoxy, alkylthio, alkylsulfinyle ou alkylsulfonyle, $R_4$ soit différent d'un groupe alcoxy,

sous forme de base libre ou de sel d'addition d'acides.

2. Un composé selon la revendication 1, dans lequel Z signifie —O— sous forme de base libre ou de sel d'addition d'acides.

3. Un composé selon la revendication 1, dans lequel $R_1$ signifie l'hydrogène, un groupe alkyle contenant de 1 à 4 atomes de carbone, hydroxyalkyle contenant au maximum 4 atomes de carbone, alcoxyalkyle contenant au maximum 6 atomes de carbone ou un groupe de formule II, où $R_5$ signifie l'hydrogène, un halogène, un groupe alkyle ou alcoxy contenant de 1 à 4 atomes de carbone, et soit i) X représente —$CH_2$— et n signifie 0, 1, 2 ou 3, soit ii) X représente —CO— et n signifie 1, 2 ou 3, soit iii) X représente —O— et n signifie 2 ou 3, $R_2$ signifie l'hydrogène, un halogène, un groupe alkyle ou alcoxy contenant de 1 à 4 atomes de carbone, $R_3$ signifie l'hydrogène, un halogène, un groupe trifluorométhyle ou alkyle, alcoxy ou alkylthio, contenant chacun de 1 à 4 atomes de carbone, $R_4$ signifie l'hydrogène, un groupe alkyle ou alcoxy contenant de 1 à 4 atomes de carbone et Z signifie —O—, avec la condition que, lorsque $R_3$ signifie un groupe trifluorométhyle, alcoxy ou alkylthio, $R_4$ soit différent d'un groupe alcoxy, sous forme de base libre ou de sel d'addition d'acides.

4. Un composé selon l'une quelconque des revendications 1, 2 et 3, dans lequel $R_1$ signifie l'hydrogène, un groupe alkyle ou un groupe de formule II, sous forme de base libre ou de sel d'addition d'acides.

5. Un composé selon l'une quelconque des revendications 1 à 4, dans lequel $R_2$ signifie l'hydrogène, un halogène ou un groupe alcoxy, sous forme de base libre ou de sel d'addition d'acides.

6. Un composé selon l'une quelconque des revendications 1 à 5, dans lequel $R_3$ signifie l'hydrogène, un halogène ou un groupe alkyle, sous forme de base libre ou de sel d'addition d'acides.

7. Un composé selon l'une quelconque des revendications 1 à 6, dans lequel $R_4$ signifie l'hydrogène, sous forme de base libre ou de sel d'addition d'acides.

8. Un composé selon la revendication 1, dans lequel $R_1$ signifie l'hydrogène, un groupe alkyle contenant de 1 à 4 atomes de carbone, hydroxyalkyle contenant au maximum 4 atomes de carbone ou alcoxyalkyle contenant au maximum 6 atomes de carbone, $R_2$ signifie l'hydrogène, un halogène, un group alkyle ou alcoxy contenant de 1 à 4 atomes de carbone, $R_3$ et $R_4$ signifient l'hydrogène et Z signifie —O—, sous forme de base libre ou sous forme de sel d'addition d'acides.

9. Un composé selon la revendication 1, qui est la 11-(4-méthyl-1-pipérazinyl)-pyrazino[2,3-b][1,5-]benzoxazépine, sous forme de base libre ou de sel d'addition d'acides.

10. Un composé selon la revendication 1, qui est la 8-chloro-11-(4-méthyl-1-pipérazinyl)-pyrazino[2,3-b][1,5]benzoxazépine, la 2-chloro-11-(4-méthyl-1-pipérazinyl)-pyrazino[2,3-b][1,5]-benzoxazépine, la 8-fluoro-11-(4-méthyl-1-pipérazinyl)-pyrazino[2,3-b][1,5]benzoxazépine, la 3-méthyl - 11 - (4 - méthyl - 1 - pipérazinyl) - pyrazino[2,3 - b][1,5]benzoxazépine, la 8 - chloro - 2 - méthyl-11-(4-méthyl-1-pipérazinyl)-pyrazino[2,3-b][1,5]benzoxazépine ou la 2,8-dichloro-11-(4-méthyl-1-pipérazinyl)-pyrazino[2,3-b][1,5]benzoxazépine, sous forme de base libre ou sous forme de sel d'addition d'acides.

11. Une composition pharmaceutique comprenant un composé de l'une quelconque des revendications 1 à 10 sous forme de base libre ou sous forme de sel d'addition d'acides acceptable du point de vue pharmaceutique, en association avec un véhicule ou un diluant pharmaceutiques.

12. Un composé tel que revendiqué à l'une quelconque des revendications 1 à 10, sous forme de base libre ou sous forme de sel d'addition d'acides acceptable du point de vue pharmaceutique, pour l'utilisation dans une méthode de traitement thérapeutique du corps humain ou animal.

13. Un composé tel que revendiqué à l'une quelconque des revendications 1 à 10, sous forme de base libre ou sous forme de sel d'addition d'acides acceptable du point de vue pharmaceutique, pour l'utilisation dans l'induction du sommeil.

14. Un procédé de préparation d'un composé de formule I, tel que défini à la revendication 1, comprenant la réaction d'un composé de formule III

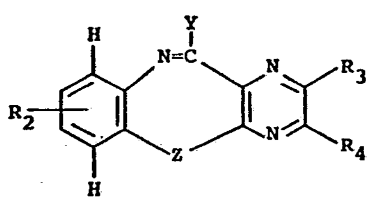

(III)

dans laquelle $R_2$, $R_3$, $R_4$ et Z sont tels que définis à la revendication 1 et Y signifie un groupe susceptible d'être éliminé, avec un composé de formule IV

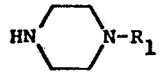

(IV)

dans laquelle $R_1$ est tel que défini à la revendication 1, et la récupération du composé résultant sous forme de base libre ou de sel d'addition d'acides.

**Patentansprüche**

1. Verbindung der Formel I,

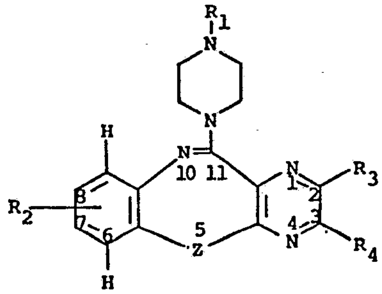

I

worin

$R_1$ für Wasserstoff, Alkyl mit 1 bis 4 Kohlenstoffatomen, Hydroxyalkyl mit höchstens 4 Kohlenstoffatomen, wobei das Hydroxyalkyl durch Alkanoyl mit 2 bis 18 Kohlenstoffatomen acyliert sein kann, Alkoxyalkyl mit höchstens 6 Kohlenstoffatomen, Cycloalkyl mit 3 bis 6 Kohlenstoffatomen, Cycloalkylalkyl mit 4 bis 7 Kohlenstoffatomen oder für eine Gruppe der Formel II steht,

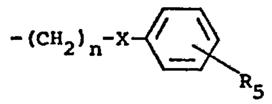

II

worin

$R_5$ für Wasserstoff, Halogen, Alkyl oder Alkoxy mit 1 bis 5 Kohlenstoffatomen, und

entweder i) X für $—CH_2—$ und
n für 0, 1, 2 oder 3, oder
ii) X für —CO— und
n für 1, 2 oder 3, oder
iii) X für —O— und
n für 2 oder 3 stehen,

$R_2$ und $R_3$ unabhängig voneinander Wasserstoff, Halogen, Trifluormethyl oder Alkyl, Alkoxy, Alkylthio, Alkylsulfinyl oder Alkylsulfonyl mit jeweils 1 bis 4 Kohlenstoffatomen bedeuten und

$R_4$ für Wasserstoff, Alkyl oder Alkoxy mit 1 bis 4 Kohlenstoffatomen steht,

Z für —O— oder —S— steht,

mit der Massgabe, dass, wenn $R_3$ Trifluormethyl, Alkoxy, Alkylthio, Alkylsulfinyl oder Alkylsulfonyl bedeutet, $R_4$ nicht für Alkoxy steht,

in Form der freien Base oder in Form ihres Säureadditionssalzes.

2. Verbindung nach Anspruch 1, worin Z für —O— steht, in Form der freien Base oder in Form ihres Säureadditionssalzes.

3. Verbindung nach Anspruch 1, worin $R_1$ für Wasserstoff, Alkyl mit 1 bis 4 Kohlenstoffatomen, Hydroxyalkyl mit höchstens 4 Kohlenstoffatomen, Alkoxyalkyl mit höchstens 6 Kohlenstoffatomen oder für eine Gruppe der Formel II steht, worin $R_5$ für Wasserstoff, Halogen, Alkyl oder Alkoxy mit 1 bis 4 Kohlenstoffatomen und entweder i) X für $—CH_2—$ und n für 0, 1, 2 oder 3, oder ii) X für —CO— und n für 1, 2 oder 3, oder iii) X für —O— und n für 2 oder 3 stehen, $R_2$ Wasserstoff, Halogen, Alkyl oder Alkoxy mit 1 bis 4 Kohlenstoffatomen, $R_3$ Wasserstoff, Halogen, Trifluormethyl oder Alkyl, Alkoxy oder Alkylthio mit jeweils 1 bis 4 Kohlenstoffatomen, $R_4$ Wasserstoff, Alkyl oder Alkoxy mit 1 bis 4 Kohlenstoffatomen bedeuten, und Z für —O— steht, mit der Massgabe, dass, wenn $R_3$ Trifluormethyl, Alkoxy oder Alkylthio bedeutet, $R_4$ nicht für Alkoxy steht, in Form der freien Base oder in Form ihres Säureadditionssalzes.

4. Verbindung nach einem der Ansprüche 1, 2 oder 3, worin $R_1$ für Wasserstoff, Alkyl oder für eine Gruppe der Formel II steht, in Form der freien Base oder in Form ihres Säureadditionssalzes.

5. Verbindung nach einem der Ansprüche 1 bis 4, worin $R_2$ für Wasserstoff, Halogen oder Alkoxy steht, in Form der freien Base oder in Form ihres Säureadditionssalzes.

6. Verbindung nach einem der Ansprüche 1 bis 5, worin $R_3$ für Wasserstoff, Halogen oder Alkyl steht, in Form der freien Base oder in Form ihres Säureadditionssalzes.

7. Verbindung nach einem Ansprüche 1 bis 6, worin $R_4$ für Wasserstoff steht, in Form der freien Base oder in Form ihres Säureadditionssalzes.

8. Verbindung nach Anspruch 1, worin $R_1$ für Wasserstoff, Alkyl mit 1 bis 4 Kohlenstoffatomen, Hydroxyalkyl mit höchstens 4 Kohlenstoffatomen oder Alkoxyalkyl mit höchstens 6 Kohlenstoffatomen, $R_2$ für Wasserstoff, Halogen, Alkyl oder Alkoxy mit 1 bis 4 Kohlenstoffatomen, $R_3$ und $R_4$ für Wasserstoff, und Z für —O— stehen, in Form der freien Base oder in Form ihres Säureadditonssalzes.

9. Als Verbindung nach Anspruch 1: 11-(4-Methyl-1-piperazinyl)-pyrazino[2,3-b][1,5]-benzoxazepin in Form der freien Base oder in Form ihres Säureadditionssalzes.

10. Als Verbindungen nach Ansrpuch 1:

8-Chlor-11-(4-methyl-1-piperazinyl)-pyrazino[2,3-b][1,5]benzoxazepin,
2-Chlor-11-(4-methyl-1-piperazinyl)-pyrazino[2,3-b][1,5]benzoxazepin,
8-Fluor-11-(4-methyl-1-piperazinyl)-pyrazino[2,3-b][1,5]benzoxazepin,
3-Methyl-11-(4-methyl-1-piperazinyl)-pyrazino[2,3-b][1,5]benzoxazepin,
8-Chlor-2-methyl-11-(4-methyl-1-piperazinyl)-pyrazino[2,3-b][1,5]benzoxazepin oder
2,8-Dichlor-11-(4-methyl-1-piperazinyl)-pyrazino[2,3-b][1,5]benzoxazepin
in Form der freien Base oder in Form ihres Säureadditionssalzes.

11. Pharmazeutisches Präparat; enthaltend eine verbindung nach einem der Ansprüche 1 bis 10 in Form der freien Base oder in Form eines pharmazeutisch verträglichen Säureadditionssalzes im Gemisch mit einem pharmazeutischen Träger- oder Verdünnungsmittel.

12. Verbindung nach einem der Ansprüche 1 bis 10 in Form der freien Base oder in Form eines

pharmazeutisch verträglichen Säureadditionssalzes zur Anwendung in einem Verfahren zur therapeutischen Behandlung des menschlichen oder tierischen Körpers.

13. Verbindung nach einem der Ansprüche 1 bis 10 in Form der freien Base oder in Form eines pharmazeutisch verträglichen Säureadditionssalzes zur Anwendung bei der Schlafeinleitung.

14. Verfahren zur Herstellung der im Anspruch 1 definierten Verbindung der Formel I dadurch gekennzeichnet, dass man eine Verbindung der Formel III,

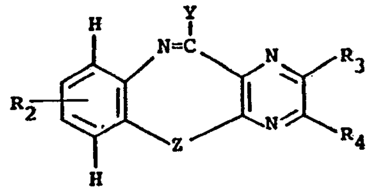

III

worin $R_2$, $R_3$, $R_4$ und Z die im Anspruch 1 angegebene Bedeutung besitzen, und Y einen abspaltbaren Rest bedeutet, mit einer Verbindung der Formel IV,

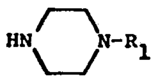

IV

worin $R_1$ die im Anspruch 1 angegebene Bedeutung besitzt, umsetzt und die erhaltene Verbindung in Form der freien Base oder in Form ihres Säureadditionssalzes gewinnt.